# EUROPEAN PATENT APPLICATION

(11) **EP 2 789 611 A1**
(43) Date of publication of application: **15.10.2014**
(21) Application number: 13162817.4
(22) Date of filing: 08.04.2013
(51) Int. Cl.: C07D 451/10

(54) **A crystalline form of tiotropium bromide**

(71) Applicant: Cerbios-Pharma S.A., 6917 Barbengo/Lugano (CH)
(72) Inventor: Mereu, Andrea, 22070 Grandate (IT); Morosoli, Moreno, 6926 Tesserete (CH); Pennè, Umberto, 6917 Barbengo/Lugano (CH); Perseghini, Mauro, 6926 Montagnola (CH)
(74) Representative: Longoni, Alessandra

(57) **Abstract**

A stable crystalline form of tiotropium bromide, and a process for its preparation with high purity.

## Description

The present invention relates to a stable crystalline form of tiotropium bromide, and to a process for its preparation with high purity.

### Background of the invention

1α,2β,4β,7β)-7-[(hydroxy-di-2-thienylacetyl)oxy]-9,9-dimethyl-3-oxa-9-azoniatricyclo[3.3.1.0^{2,4}]nonane bromide, known as tiotropium bromide (1) 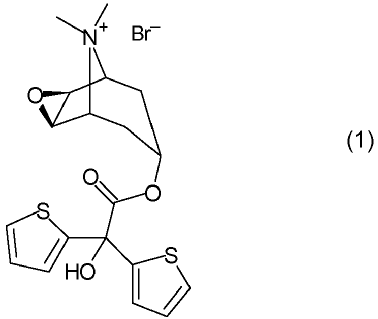 is an extremely efficient anticholinergic drug with specificity for muscarinic receptors and an extensive long-term effect. As a bronchodilator it provides therapeutic advantage in the treatment of chronic obstructive pulmonary disease (COPD) or asthma.
This pharmaceutical ingredient is used in low 18-20 microgram therapeutic dosage and is preferably administered by inhalation, and is available commercially as SPIRIVA^{®}.

A process for the preparation of tiotropium bromide (1) was first reported in EP0418716 by reaction of the corresponding cyclic tertiary amine dissolved in methylene chloride/acetonitrile with a solution of methyl bromide in acetonitrile at room temperature for 24 hours. The anhydrous crystal form obtained following the procedure described in EP0418716 is identical to the one described later in US8163913B2.

Tiotropium bromide monohydrate is disclosed in WO2002/30928 together with a method of its preparation by heating anhydrous tiotropium bromide in water.

A method of preparing anhydrous tiotropium bromide by heating tiotropium bromide monohydrate at 80-100°C under vacuum is disclosed in US6608055.

An alternative method of preparing anhydrous tiotropium bromide, disclosed in WO2007/075858, involves heating tiotropium bromide methanolate or hemi-n-butanolate or hemi-acetate in an oven at 160°C. This requires very high temperatures and specific solvates as starting material.

Several solvates were described in WO2006/117299 and WO2006/117300 by Boehringer Ingelheim and WO2007/075838 and WO2007/075858 by Sicor/Teva.

It is particularly important to develop an efficient industrial process for the preparation of tiotropium bromide on commercial scale which ensures a product with high chemical purity and a stable crystalline form.

European patent application no. 11195419.4, in the name of the same applicant, describes a continuous process for the alkylation of tertiary amines. In particular, the continuous process is useful for the quaternization of desmethyl-tiotropium.

This process does not require long reaction times and further purifications, it is high yielding and leads to a product of high purity suitable for pharmaceutical use.

One of the solvents reported in European patent application no. 11195419.4 as useful for the continuous alkylation process is N-methylpyrrolidone (NMP). However, the use of NMP alone has not been described in this previous application and no mention is made to obtaining a new crystalline form.

Surprisingly, it has been found that the alkylation reaction in pure N-methylpyrrolidone (NMP) leads to a pure tiotropium bromide solution. The desired product can be directly crystallized to produce a new crystal form.

### Summary of the invention

Object of the present invention is a new crystal from of tiotropium bromide and a process for its preparation by crystallization from a solution of tiotropium bromide in NMP.

### Brief description of the figures

Figure 1 - XRPD of the tiotropium bromide crystal form according to the invention
Figure 2 - DSC of the tiotropium bromide crystal form according to the invention
Figure 3 - TGA of the tiotropium bromide crystal form according to the invention
Figure 4 - FT-IR of the tiotropium bromide crystal form according to the invention
Figure 5 - FT-Raman of the tiotropium bromide crystal form according to the invention

### Description of the invention

Object of the present invention is a crystalline tiotropium bromide having an XRPD pattern comprising at least three peaks selected from peaks of 2[Theta] angles of about 9.44, 12.65, 15.23, 16.91, 17.71, 17.87, 18.86, 21.08, 22.35, 22.70, 22.83, 22.91, 24.09, 25.39, 25.57, 27.47, 30.62 +/- 0.2 degrees.

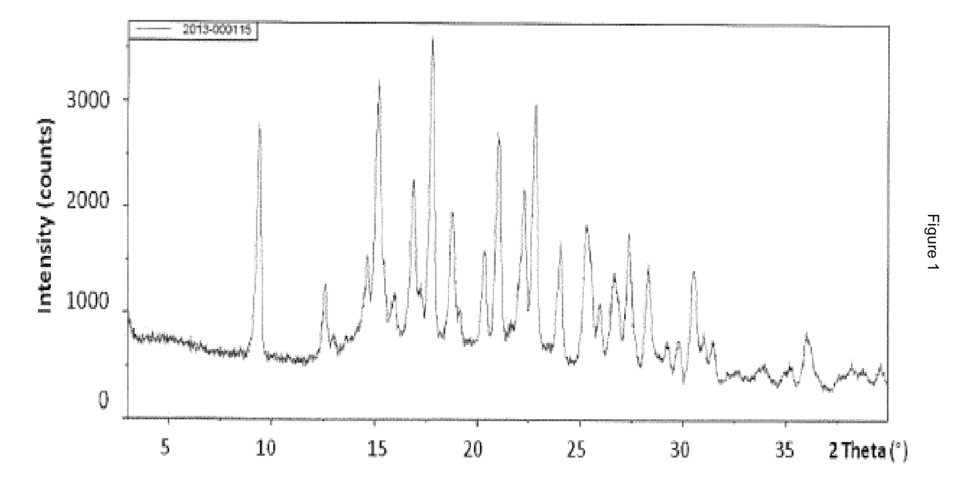

Preferably, the crystalline tiotropium bromide according to the invention has an XRPD spectrum as shown in Figure 1.

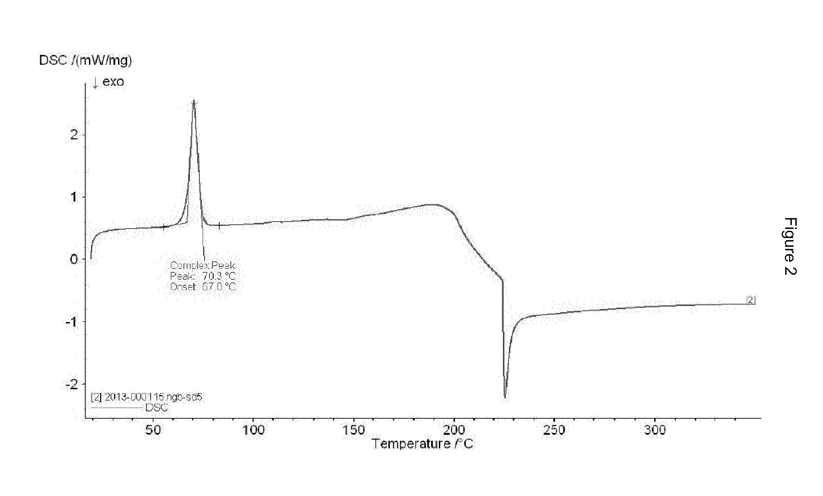

Preferably, the crystalline tiotropium bromide according to the invention has a DSC spectrum as shown in Figure 2.

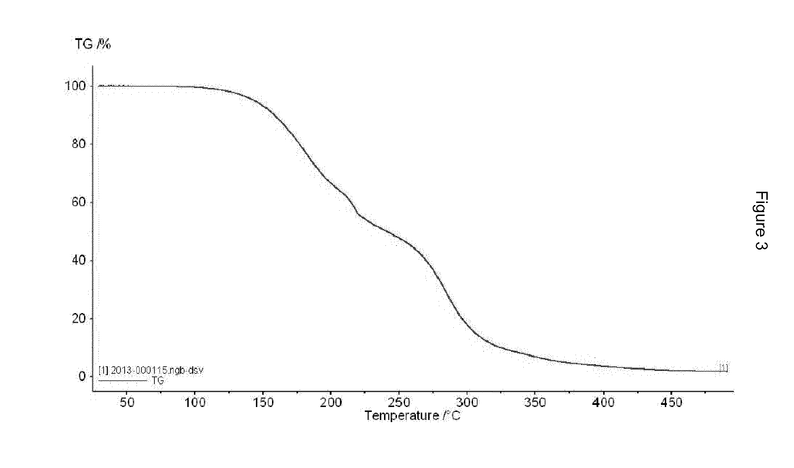

Preferably, the crystalline tiotropium bromide according to the invention has a TGA spectrum as shown in Figure 3.

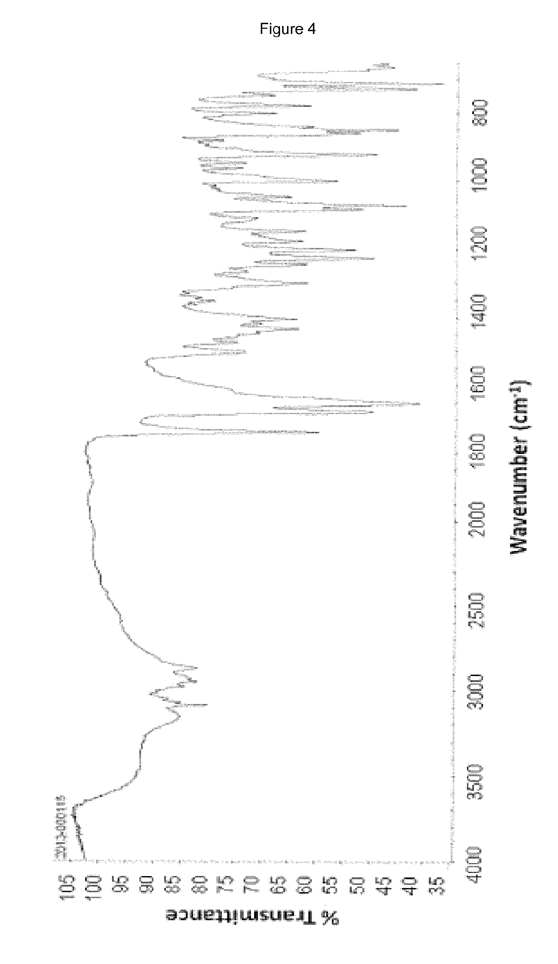

Preferably, the crystalline tiotropium bromide according to the invention has a FT-IR spectrum as shown in Figure 4.

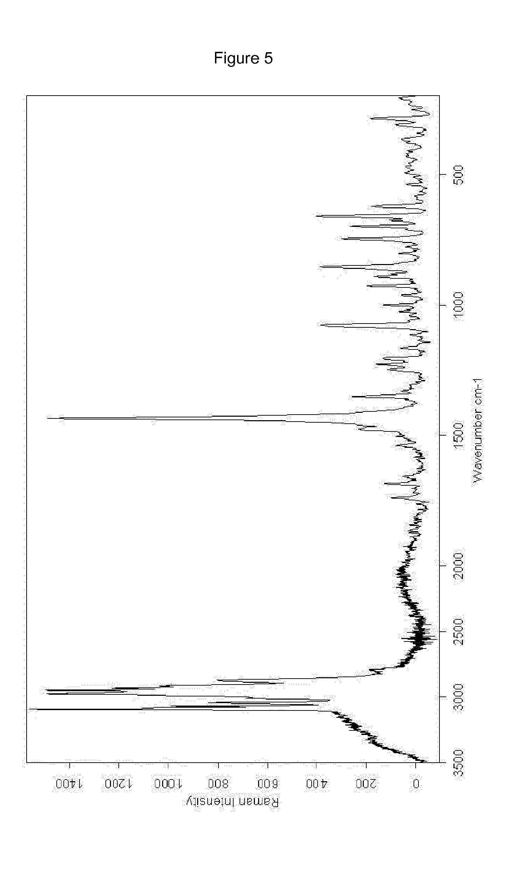

Preferably, the crystalline tiotropium bromide according to the invention has a FT-Raman spectrum as shown in Figure 5.

The crystalline tiotropium bromide according to the invention has a purity higher than 99.0% and no single impurity higher than 0.15%.

The impurity profile and X-ray spectrum did not change for at least six months at 25°C / 60% relative humidity and at 40°C/75% RH.

The crystalline tiotropium bromide according to the invention can contain traces of residual solvent (NMP). The content of NMP as residual solvent is generally lower than 3% (w/w), preferably lower than 0.3% (w/w).

Another object of this invention is a crystallization process that leads to the formation of the novel crystal form of tiotropium bromide. The crystallization process starts with a solution of tiotropium bromide in NMP. This solution can be obtained by quaternization (methylation) of desmethyl-tiotropium in NMP, or by dissolving tiotropium bromide in NMP. Crystallization occurs directly or after the optional addition of a second solvent, such as acetonitrile, tert-butylmethylether (TBME), or ethyl formate. In order to perform the crystallization, a controlled cooling profile can be used. The pure product is collected after filtration, washed with a second solvent, such as acetonitrile, TBME, or ethyl formiate, and drying under a nitrogen stream. The high purity profile and the stability of the crystalline tiotropium bromide according to the present invention make it particularly suitable for the pharmaceutical use.

### Examples

The present invention is now illustrated without limiting it by the following examples. The preparation of the starting material desmethyl-tiotropium (4) is described in Examples 1 and 2 according to Scheme 1.

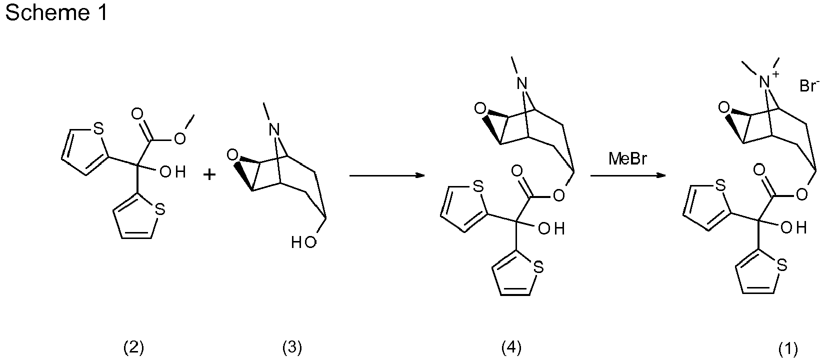

### Example 1

### Preparation of desmethyl-tiotropium (4) in NMP from scopine (3) free base

A mixture of 4.0 g (25.8 mmol) scopine (3) and 3.0 g (21.7 mmol, 0.84 eq.) anhydrous potassium carbonate in 12 mL NMP was stirred 1 h at RT. After heating to 60°C, the mixture was charged with 6.56 g (25.8 mmol, 1.00 eq.) methyl di-(2-thienyl)glycolate (2) in 8 mL NMP, followed by the addition of 1.7 g (12.3 mmol, 0.48 eq.) anhydrous potassium carbonate. The temperature was raised to 70°C and the reaction vessel was connected to the vacuum (20 mbar) for 19 h. TLC (EtOAc) indicated complete conversion of the glycolate. After cooling to 0°C, 32 mL 9% HCl were slowly added until pH-3. The dense precipitate was diluted with 20 mL water and washed twice with 10 mL toluene. The aqueous layer was cooled to 0°C and basified with 4.0 g potassium carbonate until pH-9. After 1 h, the precipitate was filtered and washed with water until the washings were neutral. Purity desmethyl-tiotropium (HPLC): 99.8%.
LCMS EI⁺ 378.0; EI⁻ 376.0.
¹H-NMR (300 MHz, d₆-DMSO): 7.50 (dd, J=5.1 Hz, 1.1, 2H), 7.25 (s, 1H), 7.07 (dd, J=3.6, 1.2 Hz, 2H), 7.00 (dd, J=5.0, 3.7 Hz, 2H), 4.95 (t, J=5.3 Hz, 1 H), 3.00 (s, 4H), 2.36 (s, 3H), 2.05-2.01 (m, 1 H), 2.00-1.95 (m, 1 H), 1.50 (s, 1 H), 1.45 (s, 1 H). ¹³C-NMR (75.5 MHz, d₆-DMSO): 170.5, 147.4, 127.1, 126.5, 126.1, 76.7, 69.1, 57.9, 56.4, 43.4, 31.6.

### Example 2

### Preparation of desmethyl-tiotropium (4) from scopine hydrochloride

A suspension of 100 g (0.522 mol) scopine hydrochloride in 350 mL DMF was charged with 72 g (0.521 mol, 1.0 eq.) anhydrous potassium carbonate. After the gas evolution completed, a solution of 159 g (0.625 mol, 1.2 eq.) methyl di-(2-thienyl)glycolate **(2)** in 300 mL DMF were added. The reaction mixture was heated to 70°C and vacuum (40 mbar) was applied. After one day, the mixture was cooled with ice and the pH lowered to 3 with 9% HCl, keeping the temperature below 25°C. The mixture was washed twice with 400 mL toluene. The aqueous layer was basified with potassium carbonate and the resulting precipitate collected by filtration and washing with water until neutral pH. The raw product was purified by crystallization from acetonitrile, affording 142 g (72%) product as light brown crystals. Purity (HPLC): 99.8%.
¹H-NMR (300 MHz, d₆-DMSO): 7.50 (dd, J=5.1 Hz, 1.1, 2H), 7.25 (s, 1H), 7.07 (dd, J=3.6, 1.2 Hz, 2H), 7.00 (dd, J=5.0, 3.7 Hz, 2H), 4.95 (t, J=5.3 Hz, 1 H), 3.00 (s, 4H), 2.36 (s, 3H), 2.05-2.01 (m, 1 H), 2.00-1.95 (m, 1 H), 1.50 (s, 1 H), 1.45 (s, 1 H). ¹³C-NMR (75.5 MHz, d₆-DMSO): 170.5, 147.4, 127.1, 126.5, 126.1, 76.7, 69.1, 57.9, 56.4, 43.4, 31.6.

### Example 3

### Preparation of tiotropium bromide (1) in NMP

To a solution of 13.2 g (39.1 mmol) desmethyl-tiotropium (4) in 30 mL NMP, 16.5 mL (115 mmol, 2.93 eq.) of a 1:1 (w/w) solution of methyl bromide in NMP were added. The mixture was stirred overnight at room temperature, whereupon a dense suspension formed. After addition of 20 mL acetonitrile, the suspension was filtered, washed with 20 mL acetonitrile, and dried with high vacuum overnight at 30°C, yielding 15.51 g of off-white powder. Residual solvents were detected by GC analysis. The XRPD pattern complied with the one shown in Figure 1.
¹H-NMR (300 MHz, d₆-DMSO): 7.52 (dd, J=5.0 Hz, 1.1, 2H), 7.41 (s, 1H), 7.13 (dd, J=3.6, 1.1 Hz, 2H), 7.01 (dd, J=5.0, 3.7 Hz, 2H), 5.12 (t, J=5.8 Hz, 1 H), 4.13 (bd, J=5.8 Hz, 2H), 3.50 (s, 2H), 3.25 (s, 3H), 3.05 (s, 3H), 2.8-2.6 (m, 2H), 1.93 (s, 1 H), 1.87 (s, 1 H).
¹³C-NMR (75.5 MHz, d₆-DMSO): 170.2, 147.1, 127.3, 126.7, 126.3, 76.8, 65.0, 64.2, 56.5, 54.1, 47.6, 28.7.

### Example 4

### Crystallization of tiotropium bromide (1) from NMP and TBME

Tiotropium bromide (0.60 g) was dissolved at 60°C by portionwise addition of 3 mL NMP. After the addition of 3 mL TBME a white crystallizate formed, which was then cooled to -10°C over 5 hours and kept at that temperature overnight. The suspension was filtered, washed twice with 2 mL and 1 mL TBME, and dried under nitrogen flow for 70 minutes. Residual solvent analysis by GC gave a NMP content lower than 3%. The XRPD pattern complied with the one shown in Figure 1.
¹H-NMR (300 MHz, d₆-DMSO): 7.52 (dd, J=5.0 Hz, 1.1, 2H), 7.41 (s, 1H), 7.13 (dd, J=3.6, 1.1 Hz, 2H), 7.01 (dd, J=5.0, 3.7 Hz, 2H), 5.12 (t, J=5.8 Hz, 1 H), 4.13 (bd, J=5.8 Hz, 2H), 3.50 (s, 2H), 3.25 (s, 3H), 3.05 (s, 3H), 2.8-2.6 (m, 2H), 1.93 (s, 1 H), 1.87 (s, 1 H).
¹³C-NMR (75.5 MHz, d₆-DMSO): 170.2, 147.1, 127.3, 126.7, 126.3, 76.8, 65.0, 64.2, 56.5, 54.1, 47.6, 28.7.

### Example 5

### Stability studies on tiotropium bromide (1)

The tiotropium bromide accelerated stability studies were performed storing the drug substance, prepared as described in the previous examples, at 40±2°C / 75±5%RH. Stability was evaluated in dark glass vial with screw cap. The frequency of the analyses performed has included five test points: 0, 7 days, 1, 3, and 6 months. Related substances amount of tiotropium bromide was measured by HPLC, following the Eur. Ph. procedure (07/2010:2420).
The results are shown in table 1.

**Table 1**

| **Related substances** | RRT | Time 0 | Time 7days | Time 30d | Time 90d | Time 180d |
|---|---|---|---|---|---|---|
| | 0.39 | | | | 0.02 | |
| Impurity A | 0.40-0.47 | **0.02** | **0.02** | **0.02** | **0.02** | **0.02** |
| Impurity B | 0.79-0.84 | **0.04** | **0.04** | **0.04** | **0.04** | **0.04** |
| | 0.98 | 0.03 | 0.03 | 0.03 | 0.02 | 0.02 |
| Tiotropium Bromide | 1.00 | **99.87** | **99.88** | **99.88** | 99.83 | 99.86 |
| | 1.21-1.35 | 0.01 | 0.01 | | 0.02 | 0.02 |
| | 1.22-1.37 | 0.02 | 0.02 | 0.03 | 0.03 | 0.03 |
| | 1.50-1.53 | 0.01 | | | 0.01 | 0.01 |
| Impurity E | 1.73 | | | | 0.01 | |
| Sum of impurities | | 0.13 | 0.12 | 0.12 | 0.17 | 0.14 |

## Claims

1. A crystalline tiotropium bromide having an XRPD pattern comprising at least three peaks selected from peaks of 2[Theta] angles of about 9.44, 12.65, 15.23, 16.91, 17.71, 17.87, 18.86, 21.08, 22.35, 22.70, 22.83, 22.91, 24.09, 25.39, 25.57, 27.47, 30.62 +/- 0.2 degrees.

2. A crystalline tiotropium bromide according to claim 1, having an XRPD spectrum as shown in Figure 1.

3. A crystalline tiotropium bromide according to anyone of claim 1-2, having an DSC spectrum as shown in Figure 2.

4. A crystalline tiotropium bromide according to anyone of claim 1-3, having an TGA spectrum as shown in Figure 3.

5. A crystalline tiotropium bromide according to anyone of claim 1-4, having an FT-IR or a FT-Raman spectrum as shown in Figures 4 and 5.

6. A crystalline tiotropium bromide according to claim 1, having a HPLC purity higher than 99.0% and no impurity higher than 0.15%.

7. A crystalline tiotropium bromide according to claim 1, containing N-methylpyrrolidone up to 3%.

8. A crystalline tiotropium bromide according to claims 1 and 7, containing N-methylpyrrolidone up to 0.3%.

9. A process for the preparation of crystalline tiotropium bromide according to claim 1 comprising the crystallization of tiotropium bromide from a N-methylpyrrolidone solution.

10. A process according to claim 9, wherein the crystallization is carried out in the presence of one or more additional solvents selected among tert-butylmethyl ether, ethyl formate and acetonitrile.
